(19) Europäisches Patentamt
European Patent Office
Office européen des brevets

(11) **EP 4 597 191 A1**

(12) **EUROPEAN PATENT APPLICATION**
published in accordance with Art. 153(4) EPC

(43) Date of publication:
06.08.2025 Bulletin 2025/32

(21) Application number: 23871986.8

(22) Date of filing: 15.09.2023

(51) International Patent Classification (IPC):
*G02B 13/00* (2006.01)    *A61B 1/00* (2006.01)
*G02B 13/16* (2006.01)    *G02B 23/26* (2006.01)

(52) Cooperative Patent Classification (CPC):
A61B 1/00; G02B 13/00; G02B 13/16; G02B 23/26

(86) International application number:
PCT/JP2023/033668

(87) International publication number:
WO 2024/070754 (04.04.2024 Gazette 2024/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 29.09.2022 JP 2022156911

(71) Applicant: **Sony Group Corporation**
**Tokyo 108-0075 (JP)**

(72) Inventor: **KIKUCHI, Naomichi**
**Tokyo 108-0075 (JP)**

(74) Representative: **D Young & Co LLP**
**3 Noble Street**
**London EC2V 7BQ (GB)**

(54) **OPTICAL SYSTEM, IMAGING DEVICE, AND ENDOSCOPE SYSTEM**

(57) The temperature characteristics of the optical system are improved. The optical system includes an image forming optical system and a separation optical system. The image forming optical system includes a first lens group and a second lens group having positive refractive power, and forms an image of light from a subject on each of a visible light imaging element and an infrared light imaging element. The separation optical system separates an optical path of light passing through the image forming optical system into an optical path of visible light to the visible light imaging element and an optical path of infrared light to the infrared light imaging element. The first lens group is an optical system including a plurality of cemented lenses formed by bonding a lens having positive refractive power and a lens having negative refractive power, in which when $\alpha p$ is an average linear expansion coefficient of the lens having positive refractive power at 100°C to 300°C and an is an average linear expansion coefficient of the lens having negative refractive power at 100°C to 300°C, the following conditional formula $|1 - \alpha p/\alpha n| < 0.2$ is satisfied.

FIG.3

AIR CONVERSION DISTANCE:BF

OVERALL LENGTH:L

**EP 4 597 191 A1**

## Description

Field

The present disclosure relates to an optical system, an imaging device, and an endoscope system.

Background

In recent years, performance of medical imaging equipment such as endoscope systems has been improved. For example, the resolution of medical imaging equipment is increased. As a result, it is possible to observe tissues that could not be handled with conventional resolution, for example, fine blood vessels, nerves, lymph nodes, or the like, and improvement in accuracy of surgery or the like is expected. In addition, medical imaging equipment capable of imaging near-infrared light in addition to visible light has been proposed. As a result, the fluorescence of the reagent can be observed, and the presence or absence of tumors such as lymph nodes or cancers and blood vessels can be easily identified. An endoscope system combining a color separation prism that separates visible light and infrared light and an image forming optical system in such medical imaging equipment has been proposed (see, for example, Patent Literature 1).

The above-described endoscope system of the related art uses an image forming optical system including two lens groups of a fixed lens group and a focus moving group using a cemented lens formed by bonding lenses having positive and negative refractive powers.

Citation List

Patent Literature

Patent Literature 1: WO 2019/187874 A

Summary

Technical Problem

However, in the above-described conventional technique, the temperature characteristics of the cemented lens to which the lenses having positive and negative refractive powers are bonded are not considered, and there is a problem that high-temperature resistance required when high-pressure steam sterilization (autoclave) or the like is applied is not sufficiently disclosed.

Therefore, the present disclosure proposes an optical system, an imaging device, and an endoscope system that improve temperature characteristics.

Solution to Problem

An optical system according to the present disclosure includes: an image forming optical system, an aperture diaphragm and a separation optical system, and when BF (air) is an air conversion distance from a vertex on a side of the separation optical system of a lens at an end portion on a side of the separation optical system among lenses having refractive power of the image forming optical system to the visible light imaging element, and L is a distance on an optical axis from the aperture diaphragm to the visible light imaging element, the following conditional formula BF (air)/L > 0.3 is satisfied. The image forming optical system includes a first lens group having positive refractive power and a second lens group configured to be movable in an optical axis direction according to a distance to a subject, and forms an image of light from the subject on each of a visible light imaging element and an infrared light imaging element. The aperture diaphragm is arranged between the subject and the image forming optical system. The separation optical system separates an optical path of light passing through the image forming optical system into an optical path of visible light to the visible light imaging element and an optical path of infrared light to the infrared light imaging element. The first lens group includes a plurality of cemented lenses formed by bonding a lens having positive refractive power and a lens having negative refractive power, and in the plurality of cemented lenses, when $\alpha p$ is an average linear expansion coefficient at 100°C to 300°C in the lens having the positive refractive power, and $\alpha n$ is an average linear expansion coefficient at 100°C to 300°C in the lens having the negative refractive power, the following conditional formula $|1 - \alpha p/\alpha n| < 0.2$ is satisfied.

Brief Description of Drawings

FIG. 1 is a diagram illustrating a configuration example of an endoscope system according to an embodiment of the present disclosure.

FIG. 2 is a diagram illustrating a configuration example of an imaging device according to an embodiment of the present disclosure.

FIG. 3 is a diagram illustrating a configuration example of an image forming optical system according to an embodiment of the present disclosure.

FIG. 4 is a diagram illustrating an example of image formation of an image forming optical system according to an embodiment of the present disclosure.

FIG. 5 is a diagram illustrating an example of shapes of lenses according to a first embodiment of the present disclosure.

FIG. 6 is a diagram illustrating an example of the shapes of the lenses according to the first embodiment of the present disclosure.

FIG. 7 is a diagram illustrating an example of characteristics of an optical system according to the first embodiment of the present disclosure.

FIG. 8 is a diagram illustrating an example of shapes of lenses according to a second embodiment of the present disclosure.

FIG. 9 is a diagram illustrating an example of the shapes of the lenses according to the second embodiment of the present disclosure.

FIG. 10 is a diagram illustrating an example of characteristics of an optical system according to the second embodiment of the present disclosure.

FIG. 11 is a diagram illustrating an example of shapes of lenses according to a third embodiment of the present disclosure.

FIG. 12 is a diagram illustrating an example of the shapes of the lenses according to the third embodiment of the present disclosure.

FIG. 13 is a diagram illustrating an example of characteristics of an optical system according to the third embodiment of the present disclosure.

FIG. 14 is a diagram illustrating an example of shapes of lenses according to a fourth embodiment of the present disclosure.

FIG. 15 is a diagram illustrating an example of the shapes of the lenses according to the fourth embodiment of the present disclosure.

FIG. 16 is a diagram illustrating an example of characteristics of an optical system according to the fourth embodiment of the present disclosure.

FIG. 17 is a diagram illustrating an example of a schematic configuration of an endoscope system.

FIG. 18 is a block diagram illustrating an example of a functional configuration of a camera and a camera control unit (CCU) illustrated in FIG. 17.

Description of Embodiments

Hereinafter, embodiments of the present disclosure will be described in detail with reference to the drawings. The description will be given in the following order. Note that, in each of the following embodiments, the same parts are denoted by the same reference signs, and redundant description will be omitted.

1. Embodiments
2. Application Examples

(1. Embodiments)

[Configuration of endoscope system]

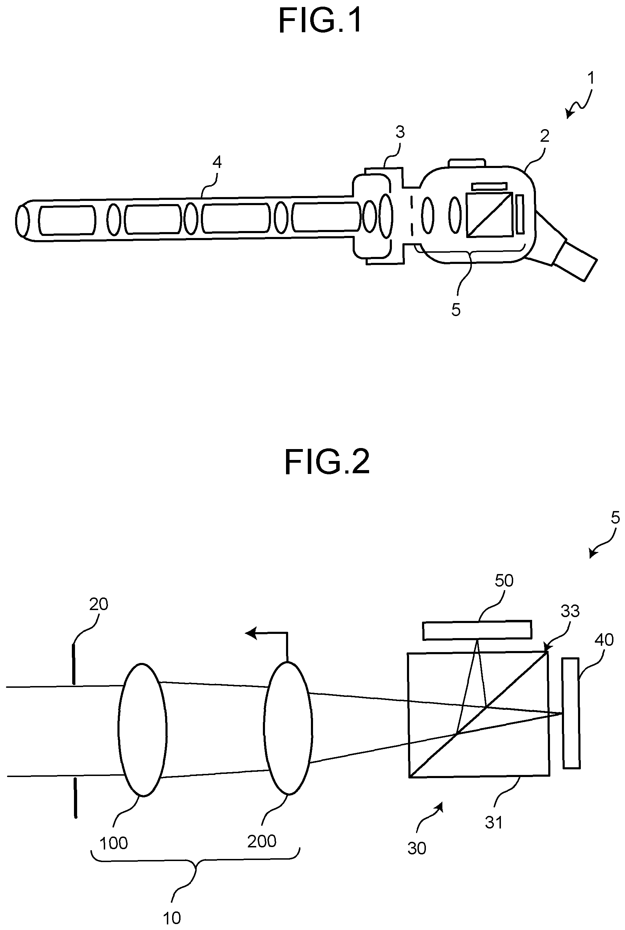

FIG. 1 is a diagram illustrating a configuration example of an endoscope system according to an embodiment of the present disclosure. The figure is a schematic diagram illustrating a configuration example of an endoscope system 1. The endoscope system 1 includes a camera head 2, a rigid endoscope 4, and an eyepiece part 3.

The rigid endoscope 4 is an endoscope in which a portion to be inserted into the body does not bend, and generates an image of tissue in the body, which is a subject. For example, the rigid endoscope 4 irradiates a subject with light, and transmits an image of reflected light from the subject by a relay lens, an optical fiber, or the like to generate (output) the image. The rigid endoscope 4 in the figure generates an infrared light image in addition to a visible light image of a subject. The infrared light image is, for example, a near-infrared fluorescence image.

The eyepiece part 3 joins the rigid endoscope 4 and the camera head 2.

The camera head 2 generates an image signal from an image of light generated by the rigid endoscope 4. The generated image signal is converted into an image by a device outside the endoscope system 1 and used for screen display or the like. The camera head 2 includes an imaging device 5.

[Configuration of imaging device]

FIG. 2 is a diagram illustrating a configuration example of the imaging device according to the embodiment of the present disclosure. The figure is a schematic diagram illustrating a configuration example of the imaging device 5. The imaging device 5 includes an image forming optical system 10, an aperture diaphragm 20, a separation optical system 30, a visible light imaging element 40, and an infrared light imaging element 50.

The aperture diaphragm 20 is arranged between the subject and the image forming optical system 10 to adjust the amount of light incident on the image forming optical system 10.

The image forming optical system 10 forms an image of light from a subject on each of the visible light imaging element 40 and the infrared light imaging element 50. The image forming optical system 10 includes a first lens group 100 and a second lens group 200. The first lens group 100 is a lens group having positive refractive power and having a fixed position in the image forming optical system 10. The second lens group 200 is a lens group configured to be movable in the optical axis direction according to the distance to the subject. Note that details of the configuration of the image forming optical system 10 will be described later.

The separation optical system 30 separates an optical path of light passing through the image forming optical system 10 into an optical path of visible light to the visible light imaging element 40 and an optical path of infrared light to the infrared light imaging element 50. The separation optical system 30 in the figure illustrates an example in which visible light travels straight and infrared light is reflected to separate these optical paths. The separation optical system 30 can include two prisms 31 bonded via a dichroic film 33. The dichroic film 33 and the prism 31 constitute a dichroic mirror. The dichroic film 33 in the figure allows visible light to travel straight and reflects infrared light. Alternatively, the dichroic film 33 may be configured to allow infrared rays to travel straight and reflect visible light. Such a prism constituting the dichroic mirror is referred to as a color separation prism.

The visible light imaging element 40 is an imaging element that is arranged at a visible light image forming position of the image forming optical system 10 and captures an image of visible light. The visible light imaging element 40 can include, for example, an imaging element in which a filter that shields infrared light is arranged. As this imaging element, for example, a complementary metal oxide semiconductor (CMOS) type imaging element can be applied.

The infrared light imaging element 50 is an imaging element that is arranged at an image forming position of infrared light of the image forming optical system 10 and performs imaging of infrared light. As the infrared light imaging element 50, for example, a CMOS imaging element in which a filter that shields visible light is arranged can be applied.

As described above, the imaging device 5 used in the endoscope system 1 has high resolution and is used to observe a fine tissue in the body. However, in order to ensure the resolution performance, it is necessary to improve manufacturability to reduce variations in optical performance and improve reliability assuming long-term use. In order to ensure high resolution performance in mass production models, methods of individual adjustment in the manufacturing process are often used. However, there are many clearances and movable parts for adjustment, and there is a concern about aged deterioration due to heat disinfection of an autoclave or the like. In addition, in the case of using the bonded lens for high resolution, there is also a risk that delamination occurs due to a difference in thermal expansion between the lenses and the bonded lens is damaged when the above-described high-temperature state by the autoclave is repeated. If scratches or fogging occurs in the lens at this time, it becomes difficult to capture an image, and surgery or the like has to be interrupted.

In view of the above technical problems, in the present disclosure, in order to ensure manufacturability and optical performance, a lens configuration in consideration of the influence of thermal expansion of an image forming optical system and a cemented lens effectively using a cemented lens will be described.

[Configuration of image forming optical system]

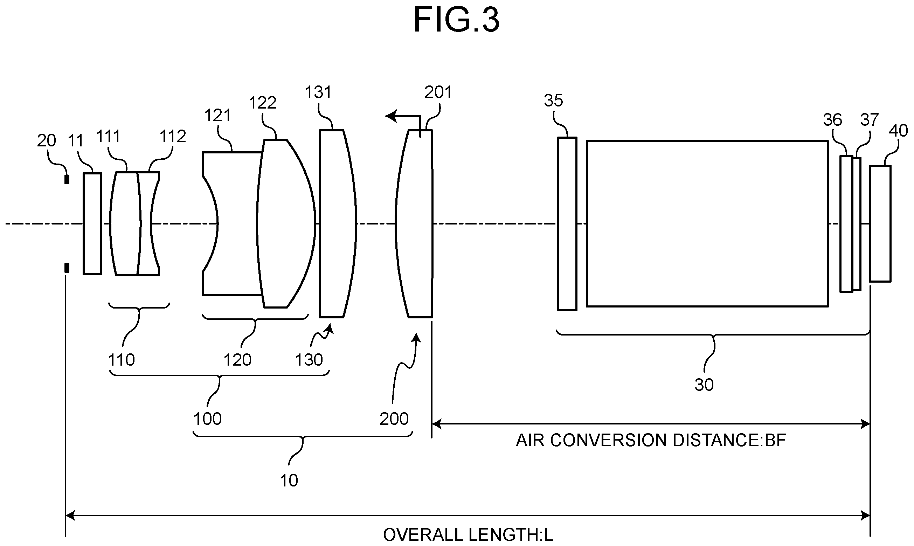

FIG. 3 is a diagram illustrating a configuration example of an image forming optical system according to the embodiment of the present disclosure. The figure is a schematic cross-sectional view illustrating a configuration example of the image forming optical system 10. Note that the aperture diaphragm 20, the separation optical system 30, and the visible light imaging element 40 are further illustrated in the figure.

Filters 35 to 37 are arranged in the separation optical system 30 in the figure. The filter 35 is arranged between the separation optical system 30 and the image forming optical system 10. The filters 36 and 37 are arranged between the separation optical system 30 and the visible light imaging element 40.

The image forming optical system 10 in the figure includes an optical element 11 in addition to the first lens group 100 and the second lens group 200 described above.

The optical element 11 is an optical element having no refractive power, and corresponds to, for example, an optical phase modulation element or a filter that transmits light of a specific wavelength. Note that a band pass filter can be applied as this filter.

The first lens group 100 in the figure illustrates an example including cemented lenses 110 and 120 and a lens 131. The cemented lenses 110 and 120 are lenses formed by bonding a lens having positive refractive power and a lens having negative refractive power. The cemented lens 110 is formed by bonding a lens 111 having positive refractive power and a lens 112 having negative refractive power. In addition, the cemented lens 120 is formed by bonding a lens 121 having negative refractive power and a lens 122 having positive refractive power. Note that the lens 131 includes a lens having positive refractive power. As illustrated in the figure, the first lens group 100 includes a plurality of cemented lenses (cemented lenses 110 and 120).

The second lens group 200 in the figure is an example including a lens 201 having positive refractive power.

In the figure, the image forming optical system 10 and the like are configured to satisfy the following conditional formula when BF (air) is an air conversion distance from a vertex of a lens (lens 201) at an end portion on the separation optical system 30 side of the lens having refractive power of the image forming optical system 10 to the visible light imaging element 40 on the separation optical system 30 side, and L is a distance on the optical axis from the aperture diaphragm 20 to the visible light imaging element 40.

$$BF(air)/L > 0.3 \qquad\qquad (1)$$

The above-described conditional formula represents a condition that the ratio of back focus (BF) to the overall lens portion length (L) in the imaging device 5 from the aperture diaphragm 20 to the light receiving surface of the visible light imaging element 40 is larger than 0.3. In a case where this conditional formula is not satisfied, the overall lens length increases, and the requirement for miniaturization is not satisfied.

In addition, in order to ensure high resolution performance and high mass productivity, it is necessary to prevent resolution performance deterioration due to decentering during manufacturing. For this reason, it is desirable that the lenses (lenses 112 and 121) having negative refractive power are always bonded to the lenses (lenses 111 and 122) having positive refractive power. It is desirable that there are two or more combinations of cemented lenses in which the positive and negative refractive power lenses are bonded.

In addition, the image forming optical system 10 needs to use a cemented lens that withstands high temperature sterilization such as an autoclave. It is desirable that the cemented lenses 110 and 120 are formed by bonding lenses that satisfy the following conditional formula when $\alpha p$ is an average linear expansion coefficient at 100°C to 300°C in a lens having positive refractive power and $\alpha n$ is an average linear expansion coefficient at 100°C to 300°C in a lens having negative refractive power.

$$|1 - \alpha p/\alpha n| < 0.2 \ (20\%) \qquad\qquad (2)$$

The conditional formula described above represents the range of the linear expansion ratio of the bonding ball. In a case where this conditional formula is not satisfied, the risk of peeling of the bonded portion increases due to the difference in stress between the lenses at the time of high temperature sterilization such as autoclave.

The image forming optical system 10 includes the second lens group 200 capable of changing an image forming position according to a distance of a subject. The second lens group 200 in the figure is an example including a single lens 201, and is lighter than a case where a plurality of lenses is arranged. Therefore, high-speed focusing can be performed. Note that, in a case where a foca method such as overall extension is adopted, the entire weight increases and a large actuator is required, which is not suitable for the demand for miniaturization. In addition, the variation of the angle of view increases, and the image frame of the rigid endoscope 4 greatly moves during frequent focus adjustment, causing the operator to observe an unnecessary change. As a result, there is a concern that stress is given to the operator. The focus moving group of the image forming optical system 10 of the present disclosure can reduce the variation in the angle of view, and can reduce the stress by making the operator feel less change in the observation range even in a case where frequent focusing is performed.

In order to implement this focus, it is desirable to conform to the following conditional formula.

$$0.8 < \Delta f/\Delta F < 0.96 \qquad\qquad (3)$$

Here, ΔF represents an arbitrary minute movement amount of the second lens group 200. In addition, Δf represents a change in the image forming position when ΔF is moved. In the above conditional formula, in a case where the ratio of the movement amount of the image forming position in the optical axis direction to the movement amount of the second lens group 200 in the optical axis direction is smaller than 0.8, the focus stroke becomes large, which is not suitable for high-speed autofocus.

In addition, when the weight of the portion of the effective diameter of the second lens group 200 is W2, it is desirable that the weight (unit: g) satisfies the following conditional formula.

$$W2 < 0.8 \tag{4}$$

In a case where this conditional formula is not satisfied, the actuator or the like has to be increased in size, so that high-speed autofocus cannot be supported.

In addition, when the specific gravity of the glass material used for the lens of the second lens group 200 is SG2, the following conditional formula is desirably satisfied.

$$SG2 \leq 3.2 \tag{5}$$

This conditional formula indicates the limitation of the glass material used for the focus group. In the imaging device 5 including the separation optical system 30, the effective diameter of the focusing ball increases, and the weight also increases. Therefore, the second lens group 200 using a glass material having a light specific gravity is desirable.

In addition, the image forming optical system 10 desirably satisfies the following conditional formula when IH1 (Near) is set as an image forming position in the visible light imaging element 40 of an image of light incident on the image forming optical system 10 of its own at an incident angle of 10 degrees when the second lens group 200 is focused at the closest distance, and IH1 (Far) is set as an image forming position in the visible light imaging element 40 of light incident on the image forming optical system 10 of its own at an incident angle of 10 degrees when the second lens group 200 is focused at the farthest distance.

$$|1 - IH1(Near)/IH1(Far)| < 0.04 \ (4\%) \tag{6}$$

The above conditional formula defines the variation (breathing) of the angle of view when the focus moves from Near to Far. In the present disclosure on the premise of active autofocus, it is desirable to satisfy the above-described conditional formula. This is to reduce the stress caused by the variation in the size of the image frame for the observer who performs surgery while viewing the image frame of the rigid endoscope 4.

In addition, like the lenses 112 and 121 of the first lens group 100 described in FIG. 3, it is desirable that two or more lenses having negative refractive power are continuously arranged, and the refractive index of the lens arranged on the subject side among these two lenses is made smaller than the refractive index of the other lens. This is to realize high resolution performance. In addition, these two lenses have concave surfaces formed on surfaces facing each other. In addition, it is desirable that the refractive index of the lens 111 having positive refractive power arranged closest to the subject side is larger. This is to further improve the resolution.

Here, when the refractive index of the lens (lens 112) having negative refractive power arranged closest to the subject side is nd (GN1) and the refractive index of the lens (lens 121) having negative refractive power arranged adjacent to the above-mentioned lens is nd (GN2), the following conditional formula is desirably satisfied.

$$nd(GN1)/nd(GN2) < 0.95 \tag{7}$$

In a case where this conditional formula is not satisfied, field curvature correction by the lens of GN1 becomes insufficient, coma aberration of the lens of GN2 increases, and imaging performance deteriorates.

In addition, when nd (GP1) is the refractive index of the lens (lens 111) having positive refractive power arranged closest to the subject side, the following conditional formula is desirably satisfied.

$$nd(GP1) > 1.85 \tag{8}$$

$$nd(GP1)/nd(GN1) > 1.2 \tag{9}$$

The conditional formula (8) indicates that it is desirable to make the refractive index of the lens of GP1 as high as possible. In a case where this conditional formula is not satisfied, a large spherical aberration occurs due to proximity to the aperture diaphragm 20, and a correction load of a lens having negative refractive power in the subsequent stage increases. In addition, it is desirable that a difference in refractive index between the lens of GP1 and the lens of GN1 is large. This is to reduce the aberration in the lens of GP1 and reduce the burden of canceling the aberration in the lens of GN1.

[Image forming example]

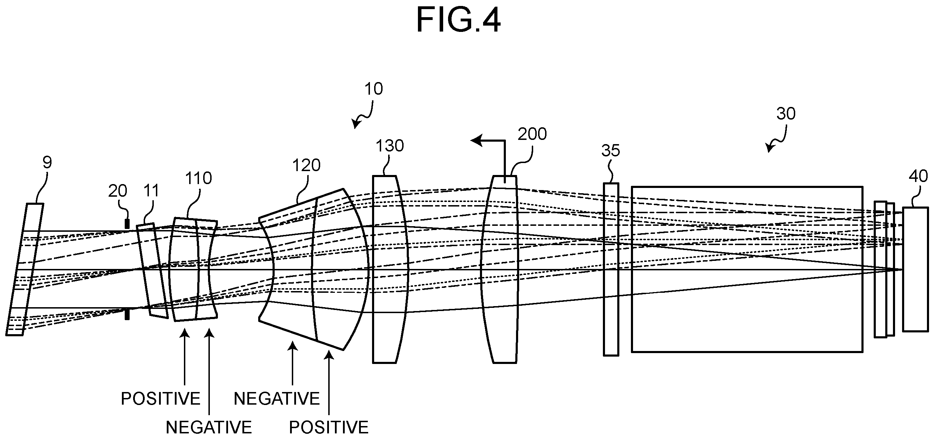

FIG. 4 is a diagram illustrating an example of image formation of the image forming optical system according to the embodiment of the present disclosure. The figure is a diagram illustrating an example of image formation of the image forming optical system 10 including a lens satisfying the above-described conditional formulas (1) to (9). Since the lens having the negative refractive power of the cemented lens 110 and the lens having the negative refractive power of the cemented lens 120 are arranged adjacent to each other, a burden on these lenses having the negative refractive power is reduced. In addition, among these lenses, since the refractive index of the lens having negative refractive power on the cemented lens 120 side is increased, correction of the spherical surface and the field curvature becomes easy. In addition, in the cemented lenses 110 and 120, by combining and bonding lenses having positive and negative refractive power, it is possible to reduce a decrease in resolution due to decentering of the lenses. In addition, furthermore, by reducing the difference between the average linear expansion coefficients of the positive and negative refractive power lenses in the cemented lenses 110 and 120, resistance under high temperature can be improved.

[First example]

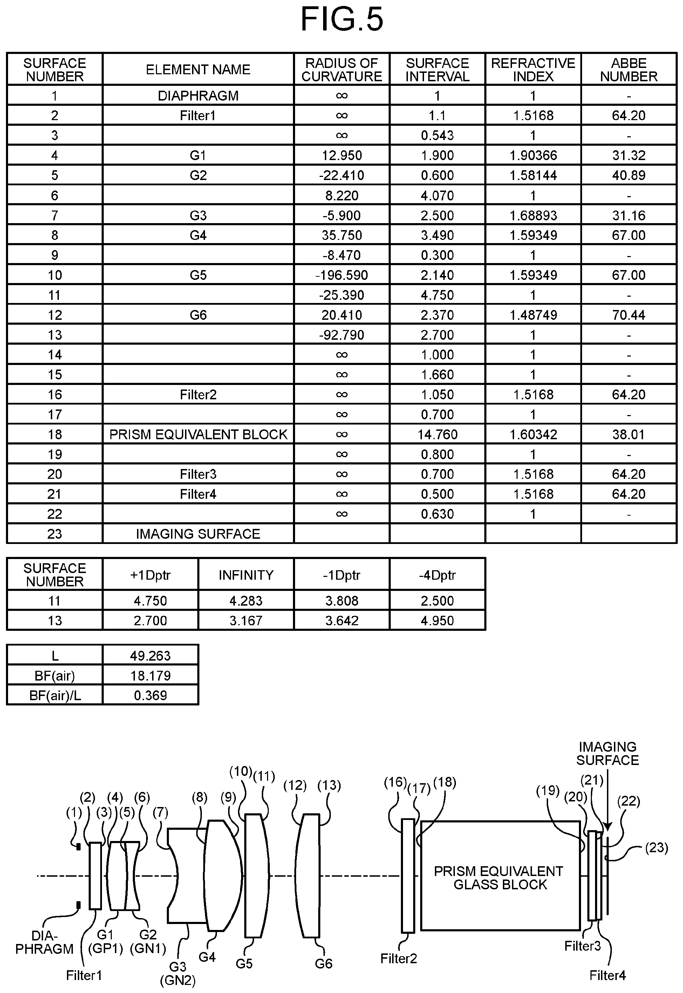

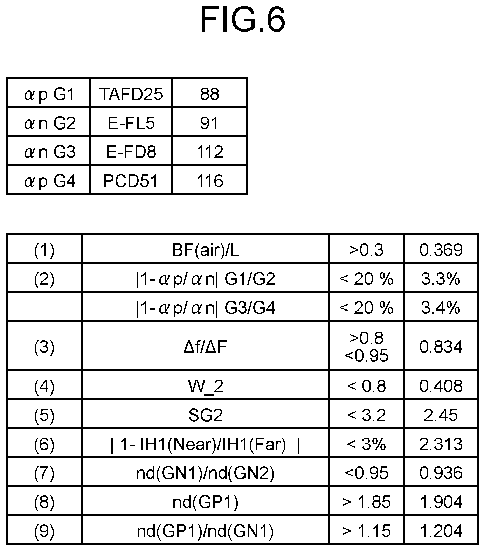

FIGS. 5 and 6 are diagrams illustrating an example of shapes of lenses according to a first embodiment of the present disclosure. In the table of FIG. 5, "surface number" represents a surface corresponding to the number in parentheses described in the lower view of FIG. 5. "Element name" represents the lens or the like illustrated in the lower view of FIG. 5. "Diaphragm" represents aperture diaphragm 20. "Filter1" represents the optical element 11. "G1" represents the lens 111. "G2" represents the lens 112. "G3" represents the lens 121. "G4" represents the lens 122. "G5" represents the lens 131. "G6" represents the lens 201. "Filter2" represents the filter 35. "Prism equivalent glass block" represents a portion of the prism 31 of the separation optical system 30. "Filter3" represents the filter 36. "Filter4" represents the filter 37. "Imaging surface" represents an imaging surface of the visible light imaging element 40.

In addition, "radius of curvature" represents the radius of curvature of the surface. "Surface interval" represents an interval between surfaces along the optical axis. "Refractive index" represents a refractive index at the d-line (wavelength: 587.6 nm) of the lens material. "Abbe number" represents the Abbe number based on the d-line. Note that values in the cases of +1Dptr, infinity, - 1Dptr, and -4Dptr are described in diopter adjustment data of another table for the surface interval (variable) of the surface numbers "11" and "13". In addition, the values of the overall lens portion length L, the air conversion distance BF (air), and BF (air)/L are described in the attached sheet.

In FIG. 6, the average linear expansion coefficients ($10^7$/K) of the lens 111 (G1), the lens 112 (G2), the lens 121 (G3), and the lens 122 (G4) at 100°C to 300°C are described in the upper table. In FIG. 6, "$\alpha$pG1", "$\alpha$pG2", "$\alpha$pG3", and "$\alpha$pG4" represent lens 111 (G1), lens 112 (G2), lens 121 (G3), and lens 122 (G4), respectively. In addition, the middle column of the table represents the names of glass materials of HOYA Corporation applicable to these lenses. In addition, the rightmost column of the table represents the average linear expansion coefficient. Note that the equivalent materials from companies such as SHOTT, OHARA, CDGM, and HIKARI can also be used for the glass materials.

In addition, item names, conditions, and simulation results corresponding to the conditional formulas (1) to (9) described above are illustrated in the lower table of FIG. 6. As described above, the optical systems illustrated in FIGS. 5 and 6 conform to the conditional formulas (1) to (9).

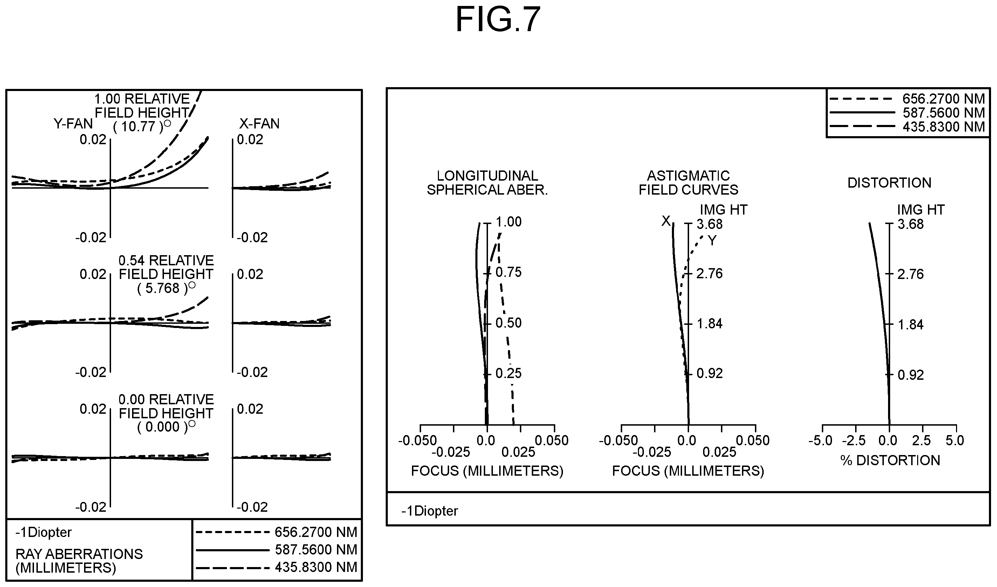

FIG. 7 is a diagram illustrating an example of characteristics of an optical system according to the first embodiment of the present disclosure. The figure is a diagram illustrating characteristics of the optical system illustrated in FIGS. 5 and 6. In addition, the figure illustrates simulation data of the ray aberration (RAY ABERRATION), the spherical aberration (LONGITUDINAL SPHERICAL ABER.), the astigmatism (ASTIGMATIC FIELD CURVES), and the distortion (DISTORTION) at the reference diopter of -1Diopter.

[Second example]

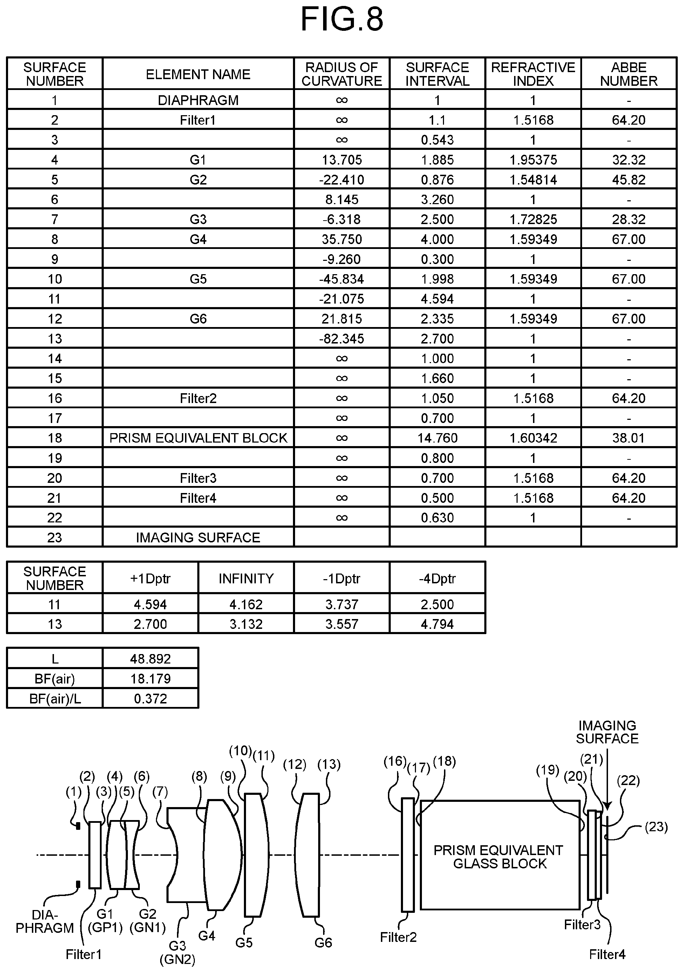

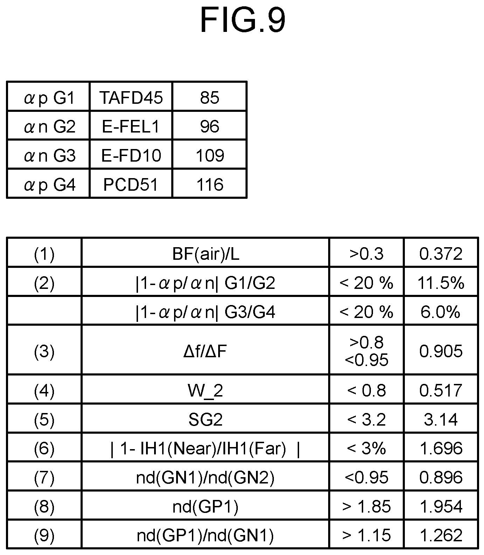

FIGS. 8 and 9 are diagrams illustrating an example of shapes of lenses according to a second embodiment of the present disclosure. In FIGS. 8 and 9, notations similar to those in FIGS. 5 and 6 are used. In addition, FIG. 9 illustrates a table similar to FIG. 7. The optical systems illustrated in FIGS. 8 and 9 conform to the conditional formulas (1) to (9).

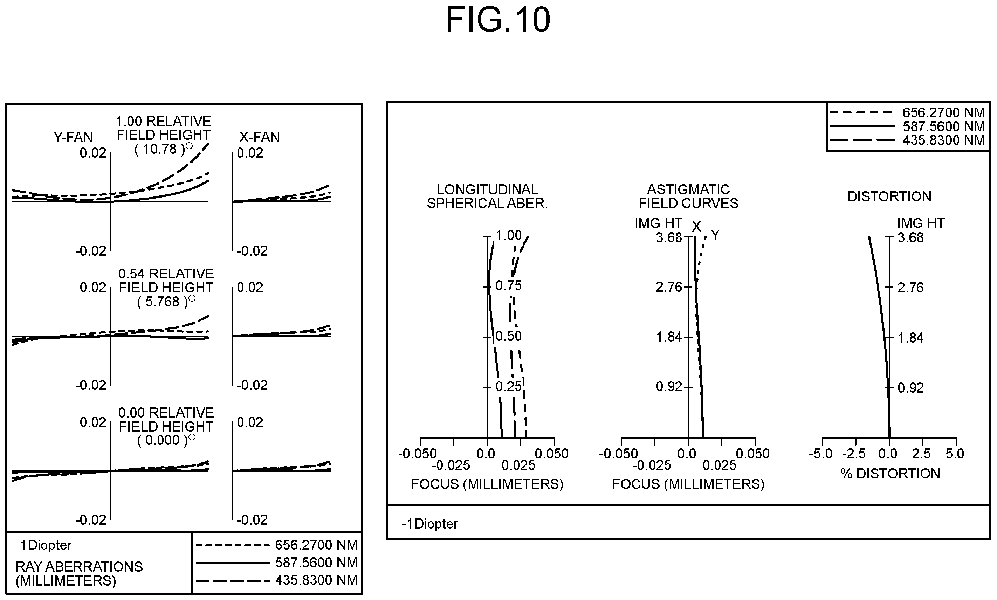

FIG. 10 is a diagram illustrating an example of characteristics of an optical system according to the second embodiment of the present disclosure. Similarly to FIG. 7, the figure is a diagram illustrating characteristics of the optical system illustrated in FIGS. 9 and 6.

[Third example]

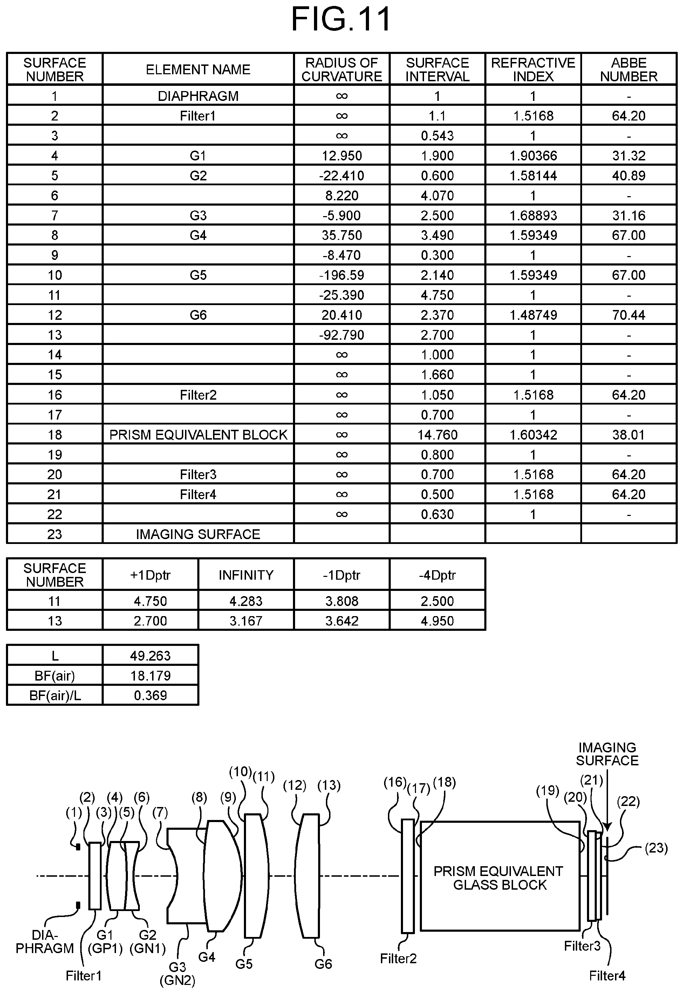

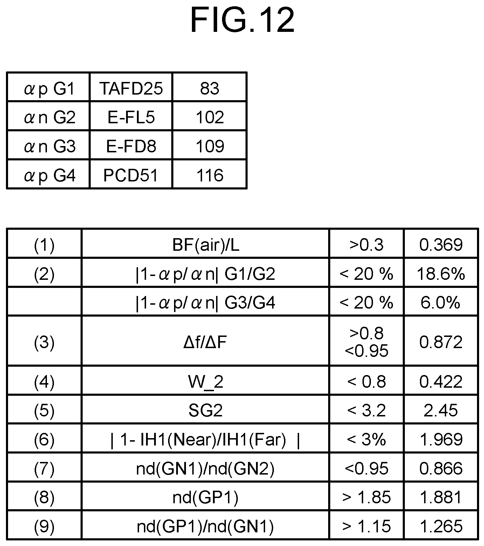

FIGS. 11 and 12 are diagrams illustrating an example of shapes of lenses according to a third embodiment of the present disclosure. In FIGS. 11 and 12, notations similar to those in FIGS. 5 and 6 are used. In addition, FIG. 12 illustrates a table similar to FIG. 7. The optical systems illustrated in FIGS. 11 and 12 conform to the conditional formulas (1) to (9).

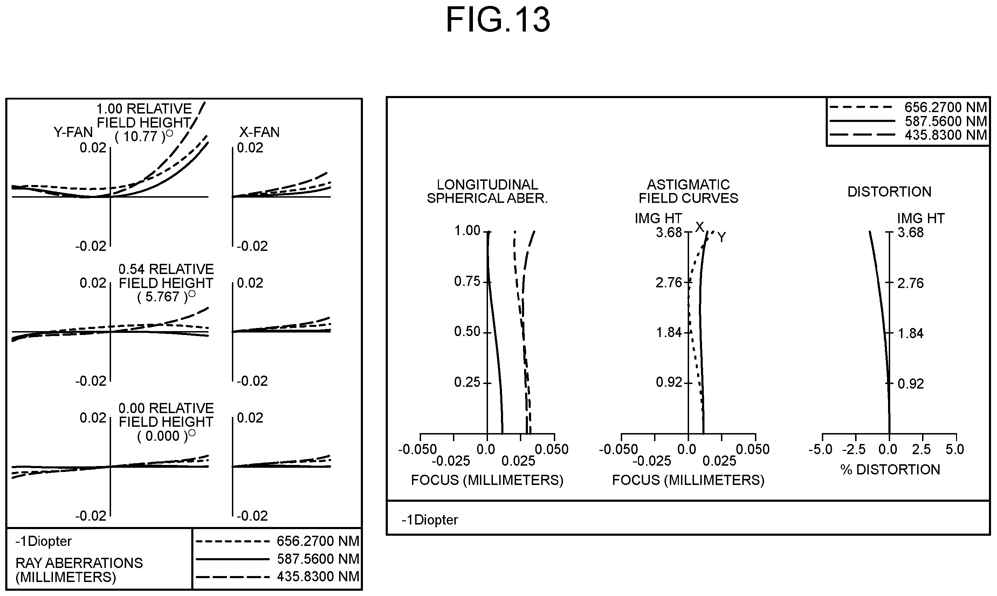

FIG. 13 is a diagram illustrating an example of characteristics of an optical system according to the third embodiment of the present disclosure. Similarly to FIG. 7, the figure is a diagram illustrating characteristics of the optical system illustrated in FIGS. 11 and 12.

[Fourth example]

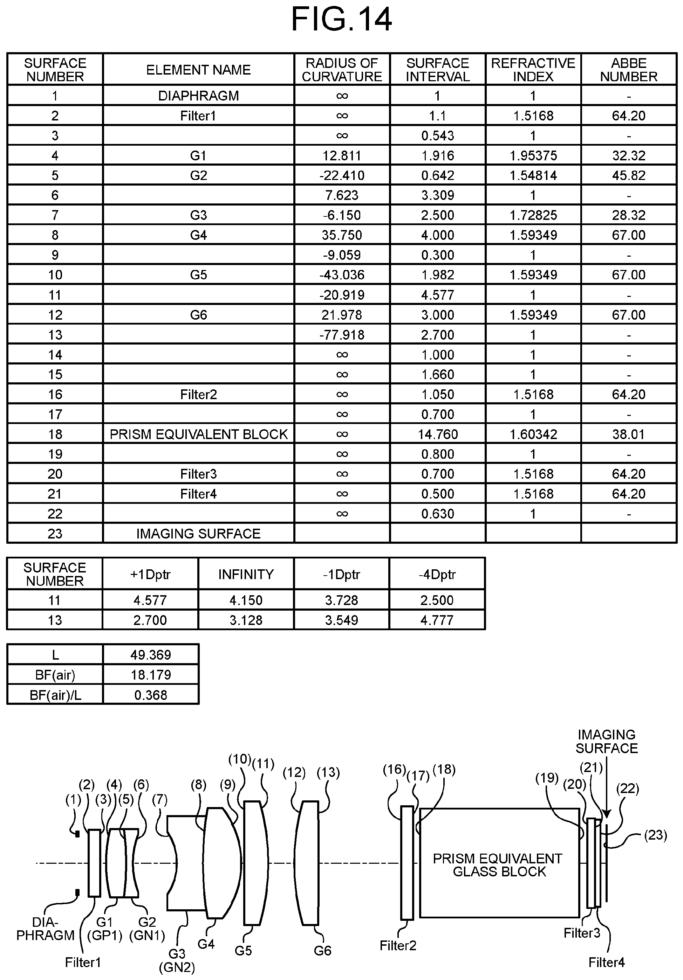

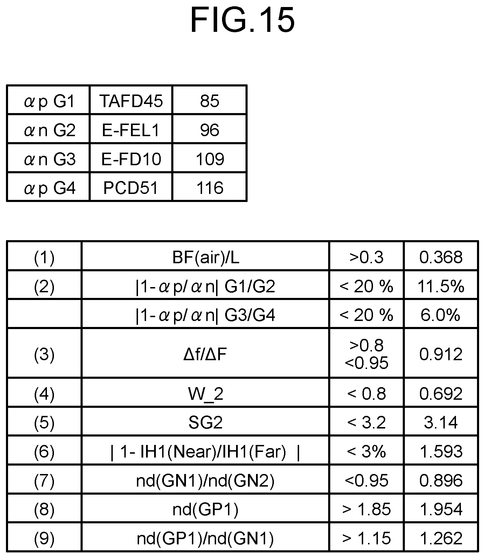

FIGS. 14 and 15 are diagrams illustrating an example of shapes of lenses according to a fourth embodiment of the present disclosure. In FIGS. 14 and 15, notations similar to those in FIGS. 5 and 6 are used. In addition, FIG. 15 illustrates a table similar to FIG. 7. The optical systems illustrated in FIGS. 14 and 15 conform to the conditional formulas (1) to (9).

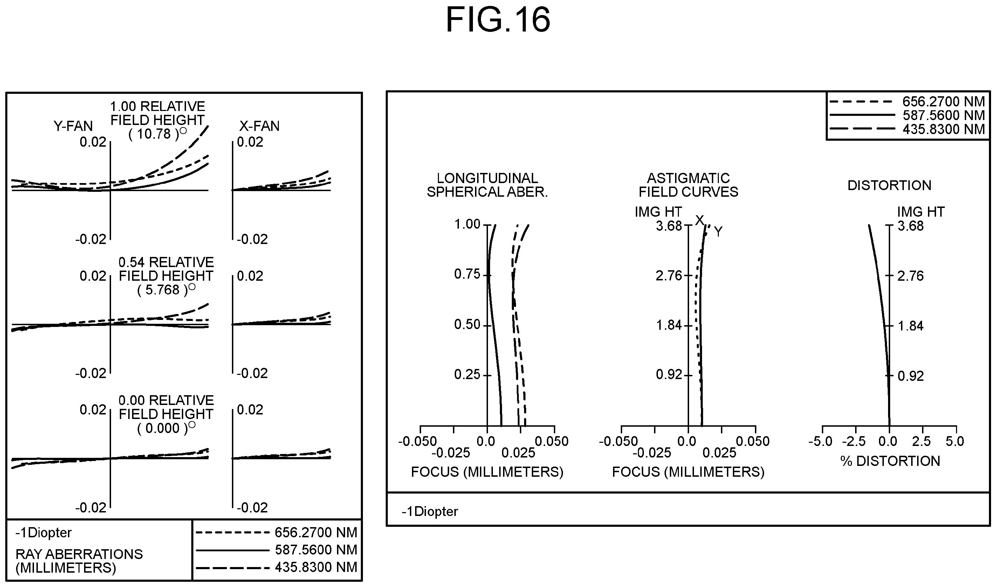

FIG. 16 is a diagram illustrating an example of characteristics of an optical system according to the fourth embodiment of the present disclosure. Similarly to FIG. 7, the figure is a diagram illustrating characteristics of the optical system illustrated in FIGS. 14 and 15.

As described above, the image forming optical systems 10 according to the embodiments of the present disclosure can improve the resistance under high temperature by reducing the difference in the average linear expansion coefficients of the cemented lenses 110 and 120 formed by bonding the lenses having positive and negative refractive powers.

(2. Application Examples)

The technology according to the present disclosure can be applied to a medical imaging system. The medical imaging system is a medical system using an imaging technology, and is, for example, an endoscope system.

[Endoscope system]

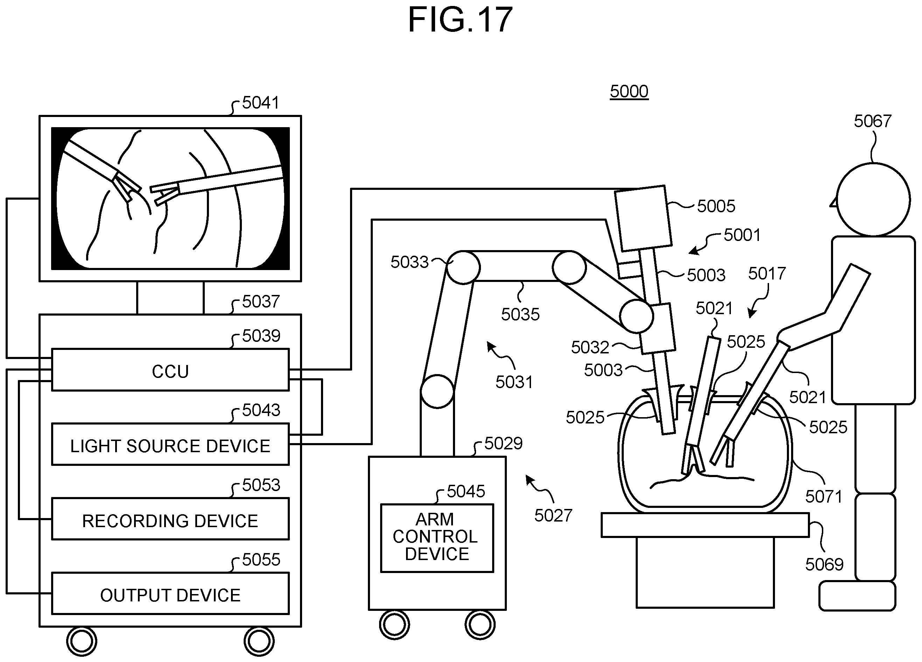

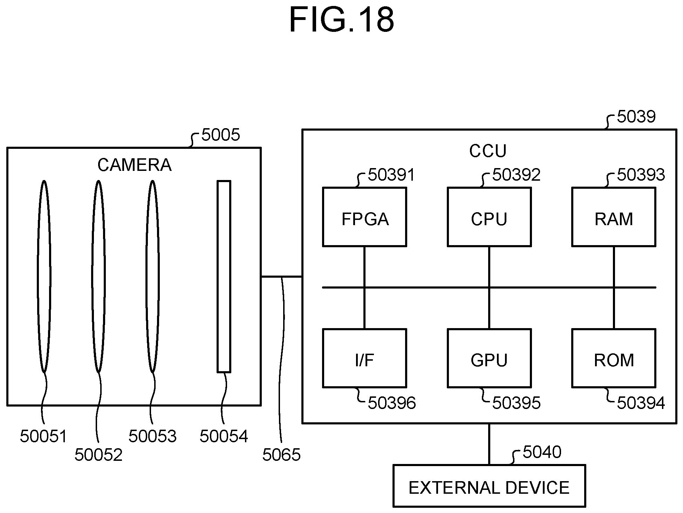

An example of the endoscope system will be described using FIGS. 17 and 18. FIG. 17 is a diagram illustrating an example of a schematic configuration of an endoscope system 5000 to which the technology according to the present disclosure is applicable. FIG. 18 is a diagram illustrating an example of a configuration of an endoscope 5001 and a camera control unit (CCU) 5039. FIG. 17 illustrates a situation where an operator (for example, a doctor) 5067 who is a participant of an operation performs the operation on a patient 5071 on a patient bed 5069 using the endoscope system 5000. As illustrated in FIG. 17, the endoscope system 5000 includes the endoscope 5001 that is a medical imaging device, the CCU 5039, a light source device 5043, a recording device 5053, an output device 5055, and a support device 5027 for supporting the endoscope 5001.

In endoscopic surgery, insertion assisting tools called trocars 5025 are punctured into the patient 5071. Then, a scope 5003 connected to the endoscope 5001 and surgical tools 5021 are inserted into a body of the patient 5071 through the trocars 5025. The surgical tools 5021 include: an energy device such as an electric scalpel; and forceps, for example.

A surgical image that is a medical image in which the inside of the body of the patient 5071 is captured by the endoscope 5001 is displayed on a display device 5041. The operator 5067 performs a procedure on a surgical target using the surgical tools 5021 while viewing the surgical image displayed on the display device 5041. The medical image is not limited to the surgical image, and may be a diagnostic image captured during diagnosis.

[Endoscope]

The endoscope 5001 is an imaging section for capturing the inside of the body of the patient 5071, and is, for example, as illustrated in FIG. 18, a camera 5005 including a condensing optical system 50051 for condensing incident light, a zooming optical system 50052 capable of optical zooming by changing a focal length of the imaging section, a focusing optical system 50053 capable of focus adjustment by changing the focal length of the imaging section, and a light receiving sensor 50054. The endoscope 5001 condenses the light through the connected scope 5003 on the light receiving sensor 50054 to generate a pixel signal, and outputs the pixel signal through a transmission system to the CCU 5039. The scope 5003 is an insertion part that includes an objective lens at a distal end and guides the light from the connected light source device 5043 into the body of the patient 5071. The scope 5003 is, for example, a rigid scope for a rigid endoscope and a flexible scope for a flexible endoscope. The scope 5003 may be a direct viewing scope or an oblique viewing scope. The pixel signal only needs to be a signal based on a signal output from a pixel, and is, for example, a raw signal or an image signal. The transmission system connecting the endoscope 5001 to the CCU 5039 may include a memory, and the memory may store parameters related to the endoscope 5001 and the CCU 5039. The memory may be disposed at a connection portion of the transmission system or on a cable. For example, the memory of the transmission system may store the parameters before shipment of the endoscope 5001 or the parameters changed when current is applied, and an operation of the endoscope may be changed based on the parameters read from the memory. A set of the camera and the transmission system may be referred to as an endoscope. The light receiving sensor 50054 is a sensor for converting the received light into the pixel signal, and is, for example, a complementary metal-oxide-semiconductor (CMOS) imaging sensor. The light receiving sensor 50054 is preferably an imaging sensor having a Bayer array capable of color imaging. The light receiving sensor 50054 is also preferably an imaging sensor having a number of pixels corresponding to a resolution of, for example, 4K (3840 horizontal pixels × 2160 vertical pixels), 8K (7680 horizontal pixels × 4320 vertical pixels), or square 4K (3840 or more horizontal pixels × 3840 or more vertical pixels). The light receiving sensor 50054 may be one sensor chip, or a plurality of sensor chips. For example, a prism may be provided to separate the incident light into predetermined wavelength bands, and the wavelength bands may be imaged by different light receiving sensors. A plurality of light receiving sensors may be provided for stereoscopic viewing. The light receiving sensor 50054 may be a sensor having a chip structure including an arithmetic processing circuit for image processing, or may be a sensor for time of flight (ToF). The transmission system is, for example, an optical fiber cable system or a wireless transmission system. The wireless transmission only needs to be capable of transmitting the pixel signal generated by the endoscope 5001, and, for example, the endoscope 5001 may be wirelessly connected to the CCU 5039, or the endoscope 5001 may be connected to the CCU 5039 via a base station in an operating room. At this time, the endoscope 5001 may transmit not only the pixel signal, but also simultaneously information (for example, a processing priority of the pixel signal and/or a synchronization signal) related to the pixel signal. In the endoscope, the scope may be integrated with the camera, and the light receiving sensor may be provided at the distal end of the scope.

[Camera control unit (CCU)]

The CCU 5039 is a control device for controlling the endoscope 5001 and the light source device 5043 connected to the CCU 5039 in an integrated manner, and is, for example, as illustrated in FIG. 18, an information processing device including a field-programmable gate array (FPGA) 50391, a central processing unit (CPU) 50392, a random access memory 50393, a read-only memory (ROM) 50394, a graphics processing unit (GPU) 50395, and an interface (I/F) 50396. The CCU 5039 may control the display device 5041, the recording device 5053, and the output device 5055 connected to the CCU 5039 in an integrated manner. The CCU 5039 controls, for example, irradiation timing, irradiation intensity, and a type of an irradiation light source of the light source device 5043. The CCU 5039 also performs image processing, such as development processing (for example, demosaic processing) and correction processing, on the pixel signal output from the endoscope 5001, and outputs the processed image signal (for example, an image) to an external device such as the display device 5041. The CCU 5039 also transmits a control signal to the endoscope 5001 to control driving of the endoscope 5001. The control signal is information on an imaging condition such as a magnification or the focal length of the imaging section. The CCU 5039 may have a function to down-convert the image, and may be configured to be capable of simultaneously outputting a higher-resolution (for example, 4K) image to the display device 5041 and a lower-resolution (for example, high-definition (HD)) image to the recording device 5053.

The CCU 5039 may be connected to external equipment (such as a recording device, a display device, an output device, and a support device) via an IP converter for converting the signal into a predetermined communication protocol (such as the Internet Protocol (IP)). The connection between the IP converter and the external equipment may be established using a wired network, or a part or the whole of the network may be established using a wireless network. For example, the IP converter on the CCU 5039 side may have a wireless communication function, and may transmit the received image to an IP switcher or an output side IP converter via a wireless communication network, such as the fifth-generation mobile communication system (5G) or the sixth-generation mobile communication system (6G).

[Light source device]

The light source device 5043 is a device capable of emitting the light having predetermined wavelength bands, and includes, for example, a plurality of light sources and a light source optical system for guiding the light of the light sources. The light sources are, for example, xenon lamps, light-emitting diode (LED) light sources, or laser diode (LD) light sources. The light source device 5043 includes, for example, the LED light sources corresponding to three respective primary colors of red (R), green (G), and blue (B), and controls output intensity and output timing of each of the light sources to emit white light. The light source device 5043 may include a light source capable of emitting special light used for special light observation, in addition to the light sources for emitting normal light for normal light observation. The special light is light having a predetermined wavelength band different from that of the normal light being light for the normal light observation, and is, for example, near-infrared light (light having a wavelength of 760 nm or longer), infrared light, blue light, or ultraviolet light. The normal light is, for example, the white light or green light. In narrow band imaging that is a kind of special light observation, blue light and green light are alternately emitted, and thus the narrow band imaging can image a predetermined tissue such as a blood vessel in a mucosal surface at high contrast using wavelength dependence of light absorption in the tissue of the body. In fluorescence observation that is a kind of special light observation, excitation light is emitted for exciting an agent injected into the tissue of the body, and fluorescence emitted by the tissue of the body or the agent as a label is received to obtain a fluorescent image, and thus the fluorescence observation can facilitate the operator to view, for example, the tissue of the body that is difficult to be viewed by the operator with the normal light. For example, in fluorescence observation using the infrared light, the infrared light having an excitation wavelength band is emitted to an agent, such as indocyanine green (ICG), injected into the tissue of the body, and the fluorescence light from the agent is received, whereby the fluorescence observation can facilitate viewing of a structure and an affected part of the tissue of the body. In the fluorescence observation, an agent (such as 5-aminolevulinic acid (5-ALA)) may be used that emits fluorescence in a red wavelength band by being excited by the special light in a blue wavelength band. The type of the irradiation light of the light source device 5043 is set by control of the CCU 5039. The CCU 5039 may have a mode of controlling the light source device 5043 and the endoscope 5001 to alternately perform the normal light observation and the special light observation. At this time, information based on a pixel signal obtained by the special light observation is preferably superimposed on a pixel signal obtained by the normal light observation. The special light observation may be an infrared light observation to observe a site inside the surface of an organ and a multi-spectrum observation utilizing hyperspectral spectroscopy. A photodynamic therapy may be incorporated.

[Recording device]

The recording device 5053 is a device for recording the pixel signal (for example, an image) acquired from the CCU 5039, and is, for example, a recorder. The recording device 5053 records an image acquired from the CCU 5039 in a hard disk drive (HDD), a Super Density Disc (SDD), and/or an optical disc. The recording device 5053 may be connected to a network in a hospital to be accessible from equipment outside the operating room. The recording device 5053 may have a down-convert function or an up-convert function.

[Display device]

The display device 5041 is a device capable of displaying the image, and is, for example, a display monitor. The display device 5041 displays a display image based on the pixel signal acquired from the CCU 5039. The display device 5041 may include a camera and a microphone to function as an input device that allows instruction input through gaze recognition, voice recognition, and gesture.

[Output device]

The output device 5055 is a device for outputting the information acquired from the CCU 5039, and is, for example, a printer. The output device 5055 prints, for example, a print image based on the pixel signal acquired from the CCU 5039 on a sheet of paper.

[Support device]

The support device 5027 is an articulated arm including a base 5029 including an arm control device 5045, an arm 5031 extending from the base 5029, and a holding part 5032 mounted at a distal end of the arm 5031. The arm control device 5045 includes a processor such as a CPU, and operates according to a predetermined computer program to control driving of the arm 5031. The support device 5027 uses the arm control device 5045 to control parameters including, for example, lengths of links 5035 constituting the arm 5031 and rotation angles and torque of joints 5033 so as to control, for example, the position and attitude of the endoscope 5001 held by the holding part 5032. This control can change the position or attitude of the endoscope 5001 to a desired position or attitude, makes it possible to insert the scope 5003 into the patient 5071, and can change the observed area in the body. The support device 5027 functions as an endoscope support arm for supporting the endoscope 5001 during the operation. Thus, the support device 5027 can play a role of a scopist who is an assistant holding the endoscope 5001. The support device 5027 may be controlled using an autonomous control method by the arm control device 5045, or may be controlled using a control method in which the arm control device 5045 performs the control based on input of a user. The control method may be, for example, a master-slave method in which the support device 5027 serving as a slave device (replica device) that is a patient cart is controlled based on a movement of a master device (primary device) that is an operator console at a hand of the user. The support device 5027 may be remotely controllable from outside the operating room.

The example of the endoscope system 5000 to which the technology according to the present disclosure is applicable has been described above. Systems to which the technology according to the present disclosure is applicable are not limited to such examples. For example, the support device 5027 can support, at the distal end thereof, another observation device or another surgical tool instead of the endoscope 5001. Examples of the other applicable observation device include forceps, tweezers, a pneumoperitoneum tube for pneumoperitoneum, and an energy treatment tool for incising a tissue or sealing a blood vessel by cauterization. By using the support device to support the observation device or the surgical tool described above, the position thereof can be more stably fixed and the load of the medical staff can be lower than in a case where the medical staff manually supports the observation device or the surgical tool. The technology according to the present disclosure may be applied to a support device for supporting such a component other than the endoscope.

The technology according to the present disclosure can be suitably applied to the camera 5005 among the configurations described above. By applying the technology according to the present disclosure to the camera 5005, it is possible to improve the resistance of the endoscope 5001 under high temperature conditions such as high temperature steam sterilization.

In addition, each component of each device illustrated in the drawings is functionally conceptual, and is not necessarily physically configured as illustrated in the drawings. That is, a specific form of distribution and integration of each device is not limited to the illustrated forms, and all or a part thereof can be functionally or physically distributed and integrated in an arbitrary unit according to various loads, usage conditions, and the like. Note that this configuration by distribution and integration may be performed dynamically.

Although the embodiments of the present disclosure have been described above, the technical scope of the present disclosure is not limited to the above-described embodiments as it is, and various modifications can be made without departing from the gist of the present disclosure. In addition, components of different embodiments and modifications may be appropriately combined.

Note that the effects described in the present specification are merely examples and are not limited, and other effects may be provided.

Note that the present technology can also have the following configurations.

(1) An optical system comprising:

an image forming optical system that includes a first lens group having positive refractive power and a second lens group configured to be movable in an optical axis direction according to a distance to a subject, and forms an image of light from the subject on each of a visible light imaging element and an infrared light imaging element;
an aperture diaphragm arranged between the subject and the image forming optical system; and
a separation optical system that separates an optical path of light passing through the image forming optical system into an optical path of visible light to the visible light imaging element and an optical path of infrared light to the infrared light imaging element,
wherein when BF (air) is an air conversion distance from a vertex on a side of the separation optical system of a lens at an end portion on a side of the separation optical system among lenses having refractive power of the image forming optical system to the visible light imaging element, and L is a distance on an optical axis from the aperture diaphragm to the visible light imaging element, the following conditional formula

$$BF\ (air)/L > 0.3$$

is satisfied,
the first lens group includes a plurality of cemented lenses formed by bonding a lens having positive refractive power and a lens having negative refractive power, and
in the plurality of cemented lenses, when $\alpha p$ is an average linear expansion coefficient at 100°C to 300°C in the lens having the positive refractive power, and $\alpha n$ is an average linear expansion coefficient at 100°C to 300°C in the lens having the negative refractive power, the following conditional formula

$$|1 - \alpha p/\alpha n| < 0.2$$

is satisfied.

(2) The optical system according to the above (1), wherein the separation optical system includes a dichroic mirror.
(3) The optical system according to the above (1) or (2), wherein the second lens group is configured such that a ratio of a movement amount in an optical axis direction of an image forming position to a movement amount in the optical axis direction of the second lens group is a value larger than 0.8 and less than 0.96.
(4) The optical system according to any one of the above (1) to (3), wherein the second lens group is configured such that a weight of a portion of an effective diameter of the second lens group is less than 0.8 g.

(5) The optical system according to the above (1), wherein a specific gravity of a glass material used in the second lens group is 3.2 or less.

(6) The optical system according to any one of the above (1) to (5), wherein in the image forming optical system, when IH1 (Near) is an image forming position in the visible light imaging element of an image of light incident on the image forming optical system at an incident angle of 10 degrees when the second lens group is focused at the closest distance, and IH1 (Far) is an image forming position in the visible light imaging element of light incident on the image forming optical system at an incident angle of 10 degrees when the second lens group is focused at the farthest distance, the following conditional formula

$$|1 - IH1(Near)/IH1(Far)| < 0.04$$

is satisfied.

(7) The optical system according to any one of the above (1) to (6), wherein in the image forming optical system, when nd (GP1) and nd (GP1) are refractive indexes of the lens having the positive refractive power and the lens having the negative refractive power constituting the cemented lens at the end portion on a side of the subject among the plurality of cemented lenses, respectively, the following conditional formulas

$$nd (GP1) > 1.85,$$

and

$$nd (GP1)/nd (GN1) > 1.2$$

are satisfied.

(8) The optical system according to any one of the above (1) to (7), wherein the image forming optical system includes two cemented lenses in which lenses having negative refractive power are arranged adjacent to each other, and the lenses having the negative refractive power of the two cemented lenses have concave surfaces formed on surfaces facing each other.

(9) The optical system according to any one of the above (1) to (8), further comprising an optical element arranged between the aperture diaphragm and the image forming optical system.

(10) The optical system according to the above (9), wherein the optical element is an optical phase modulation element.

(11) The optical system according to the above (9), wherein the optical element is a filter that transmits light of a specific wavelength.

(12) An imaging device comprising:

a visible light imaging element;
an infrared light imaging element;
an image forming optical system that includes a first lens group having positive refractive power and a second lens group configured to be movable in an optical axis direction according to a distance to a subject, and forms an image of light from the subject on each of the visible light imaging element and the infrared light imaging element;
an aperture diaphragm arranged between the subject and the image forming optical system; and
a separation optical system that separates an optical path of light passing through the image forming optical system into an optical path of visible light to the visible light imaging element and an optical path of infrared light to the infrared light imaging element,
wherein when BF (air) is an air conversion distance from a vertex on a side of the separation optical system of a lens at an end portion on a side of the separation optical system among lenses having refractive power of the image forming optical system to the visible light imaging element, and L is a distance on an optical axis from the aperture diaphragm to the visible light imaging element, the following conditional formula

$$BF (air)/L > 0.3$$

is satisfied,
the first lens group includes a plurality of cemented lenses formed by bonding a lens having positive refractive power and a lens having negative refractive power, and
in the plurality of cemented lenses, when $\alpha$p is an average linear expansion coefficient at 100°C to 300°C in the lens having the positive refractive power, and $\alpha$n is an average linear expansion coefficient at 100°C to 300°C in the lens having the negative refractive power, the following conditional formula

$$|1 - \alpha p/\alpha n| < 0.2$$

is satisfied.

(13) An endoscope system comprising:

a rigid endoscope unit that generates a visible light image and an infrared light image of a subject;
a visible light imaging element;
an infrared light imaging element; and
an optical system connected to the rigid endoscope unit to form the visible light image on the visible light imaging element and form the infrared light image on the infrared light imaging element,
the optical system including:

an image forming optical system that includes a first lens group having positive refractive power and a second lens group configured to be movable in an optical axis direction according to a distance to a subject, and forms an image of light from the subject on each of the visible light imaging element and the infrared light imaging element;
an aperture diaphragm arranged between the subject and the image forming optical system; and
a separation optical system that separates an optical path of light passing through the image forming optical system into an optical path of visible light to the visible light imaging element and an optical path of infrared light to the infrared light imaging element,
wherein when BF (air) is an air conversion distance from a vertex on a side of the separation optical system of a lens at an end portion on a side of the separation optical system among lenses having refractive power of the image forming optical system to the visible light imaging element, and L is a distance on an optical axis from the aperture diaphragm to the visible light imaging element, the following conditional formula

$$BF\ (air)/L > 0.3$$

is satisfied,
the first lens group includes a plurality of cemented lenses formed by bonding a lens having positive refractive power and a lens having negative refractive power, and
in the plurality of cemented lenses, when $\alpha$p is an average linear expansion coefficient at 100°C to 300°C in the lens having the positive refractive power, and $\alpha$n is an average linear expansion coefficient at 100°C to 300°C in the lens having the negative refractive power, the following conditional formula

$$|1 - \alpha p/\alpha n| < 0.2$$

is satisfied.

Reference Signs List

1 ENDOSCOPE SYSTEM
4 RIGID ENDOSCOPE
5 IMAGING DEVICE
10 IMAGE FORMING OPTICAL SYSTEM
11 OPTICAL ELEMENT
30 SEPARATION OPTICAL SYSTEM
31 PRISM
33 DICHROIC FILM
35 to 37 FILTER
40 VISIBLE LIGHT IMAGING ELEMENT
50 INFRARED LIGHT IMAGING ELEMENT

100 FIRST LENS GROUP
110, 120 CEMENTED LENS
111, 112, 121, 122, 131, 201 LENS
200 SECOND LENS GROUP
5001 ENDOSCOPE
5005 CAMERA

**Claims**

1. An optical system comprising:

   an image forming optical system that includes a first lens group having positive refractive power and a second lens group configured to be movable in an optical axis direction according to a distance to a subject, and forms an image of light from the subject on each of a visible light imaging element and an infrared light imaging element;
   an aperture diaphragm arranged between the subject and the image forming optical system; and
   a separation optical system that separates an optical path of light passing through the image forming optical system into an optical path of visible light to the visible light imaging element and an optical path of infrared light to the infrared light imaging element,
   wherein when BF (air) is an air conversion distance from a vertex on a side of the separation optical system of a lens at an end portion on a side of the separation optical system among lenses having refractive power of the image forming optical system to the visible light imaging element, and L is a distance on an optical axis from the aperture diaphragm to the visible light imaging element, the following conditional formula

   $$BF\ (air)/L > 0.3$$

   is satisfied,
   the first lens group includes a plurality of cemented lenses formed by bonding a lens having positive refractive power and a lens having negative refractive power, and
   in the plurality of cemented lenses, when $\alpha p$ is an average linear expansion coefficient at 100°C to 300°C in the lens having the positive refractive power, and $\alpha n$ is an average linear expansion coefficient at 100°C to 300°C in the lens having the negative refractive power, the following conditional formula

   $$|1 - \alpha p/\alpha n| < 0.2$$

   is satisfied.

2. The optical system according to claim 1, wherein the separation optical system includes a dichroic mirror.

3. The optical system according to claim 1, wherein the second lens group is configured such that a ratio of a movement amount in an optical axis direction of an image forming position to a movement amount in the optical axis direction of the second lens group is a value larger than 0.8 and less than 0.96.

4. The optical system according to claim 1, wherein the second lens group is configured such that a weight of a portion of an effective diameter of the second lens group is less than 0.8 g.

5. The optical system according to claim 1, wherein a specific gravity of a glass material used in the second lens group is 3.2 or less.

6. The optical system according to claim 1, wherein in the image forming optical system, when IH1 (Near) is an image forming position in the visible light imaging element of an image of light incident on the image forming optical system at an incident angle of 10 degrees when the second lens group is focused at the closest distance, and IH1 (Far) is an image forming position in the visible light imaging element of light incident on the image forming optical system at an incident angle of 10 degrees when the second lens group is focused at the farthest distance, the following conditional formula

   $$|1 - IH1(Near)/IH1(Far)| < 0.04$$

is satisfied.

7. The optical system according to claim 1, wherein in the image forming optical system, when nd (GP1) and nd (GP1) are refractive indexes of the lens having the positive refractive power and the lens having the negative refractive power constituting the cemented lens at the end portion on a side of the subject among the plurality of cemented lenses, respectively, the following conditional formulas

$$nd\ (GP1)\ >\ 1.85,$$

and

$$nd\ (GP1)/nd\ (GN1)\ >\ 1.2$$

are satisfied.

8. The optical system according to claim 1, wherein the image forming optical system includes two cemented lenses in which lenses having negative refractive power are arranged adjacent to each other, and the lenses having the negative refractive power of the two cemented lenses have concave surfaces formed on surfaces facing each other.

9. The optical system according to claim 1, further comprising an optical element arranged between the aperture diaphragm and the image forming optical system.

10. The optical system according to claim 9, wherein the optical element is an optical phase modulation element.

11. The optical system according to claim 9, wherein the optical element is a filter that transmits light of a specific wavelength.

12. An imaging device comprising:

a visible light imaging element;
an infrared light imaging element;
an image forming optical system that includes a first lens group having positive refractive power and a second lens group configured to be movable in an optical axis direction according to a distance to a subject, and forms an image of light from the subject on each of the visible light imaging element and the infrared light imaging element;
an aperture diaphragm arranged between the subject and the image forming optical system; and
a separation optical system that separates an optical path of light passing through the image forming optical system into an optical path of visible light to the visible light imaging element and an optical path of infrared light to the infrared light imaging element,
wherein when BF (air) is an air conversion distance from a vertex on a side of the separation optical system of a lens at an end portion on a side of the separation optical system among lenses having refractive power of the image forming optical system to the visible light imaging element, and L is a distance on an optical axis from the aperture diaphragm to the visible light imaging element, the following conditional formula

$$BF\ (air)/L\ >\ 0.3$$

is satisfied,
the first lens group includes a plurality of cemented lenses formed by bonding a lens having positive refractive power and a lens having negative refractive power, and
in the plurality of cemented lenses, when $\alpha p$ is an average linear expansion coefficient at 100°C to 300°C in the lens having the positive refractive power, and $\alpha n$ is an average linear expansion coefficient at 100°C to 300°C in the lens having the negative refractive power, the following conditional formula

$$|1\ -\ \alpha p/\alpha n|\ <\ 0.2$$

is satisfied.

**13.** An endoscope system comprising:

a rigid endoscope unit that generates a visible light image and an infrared light image of a subject;
a visible light imaging element;
an infrared light imaging element; and
an optical system connected to the rigid endoscope unit to form the visible light image on the visible light imaging element and form the infrared light image on the infrared light imaging element,
the optical system including:

an image forming optical system that includes a first lens group having positive refractive power and a second lens group configured to be movable in an optical axis direction according to a distance to a subject, and forms an image of light from the subject on each of the visible light imaging element and the infrared light imaging element;
an aperture diaphragm arranged between the subject and the image forming optical system; and
a separation optical system that separates an optical path of light passing through the image forming optical system into an optical path of visible light to the visible light imaging element and an optical path of infrared light to the infrared light imaging element,
wherein when BF (air) is an air conversion distance from a vertex on a side of the separation optical system of a lens at an end portion on a side of the separation optical system among lenses having refractive power of the image forming optical system to the visible light imaging element, and L is a distance on an optical axis from the aperture diaphragm to the visible light imaging element, the following conditional formula

$$BF\ (air)/L > 0.3$$

is satisfied,
the first lens group includes a plurality of cemented lenses formed by bonding a lens having positive refractive power and a lens having negative refractive power, and
in the plurality of cemented lenses, when $\alpha p$ is an average linear expansion coefficient at 100°C to 300°C in the lens having the positive refractive power, and $\alpha n$ is an average linear expansion coefficient at 100°C to 300°C in the lens having the negative refractive power, the following conditional formula

$$|1 - \alpha p/\alpha n| < 0.2$$

is satisfied.

# FIG.1

# FIG.2

FIG.3

# FIG.4

# FIG.5

| SURFACE NUMBER | ELEMENT NAME | RADIUS OF CURVATURE | SURFACE INTERVAL | REFRACTIVE INDEX | ABBE NUMBER |
|---|---|---|---|---|---|
| 1 | DIAPHRAGM | ∞ | 1 | 1 | - |
| 2 | Filter1 | ∞ | 1.1 | 1.5168 | 64.20 |
| 3 | | ∞ | 0.543 | 1 | - |
| 4 | G1 | 12.950 | 1.900 | 1.90366 | 31.32 |
| 5 | G2 | -22.410 | 0.600 | 1.58144 | 40.89 |
| 6 | | 8.220 | 4.070 | 1 | - |
| 7 | G3 | -5.900 | 2.500 | 1.68893 | 31.16 |
| 8 | G4 | 35.750 | 3.490 | 1.59349 | 67.00 |
| 9 | | -8.470 | 0.300 | 1 | - |
| 10 | G5 | -196.590 | 2.140 | 1.59349 | 67.00 |
| 11 | | -25.390 | 4.750 | 1 | - |
| 12 | G6 | 20.410 | 2.370 | 1.48749 | 70.44 |
| 13 | | -92.790 | 2.700 | 1 | - |
| 14 | | ∞ | 1.000 | 1 | - |
| 15 | | ∞ | 1.660 | 1 | - |
| 16 | Filter2 | ∞ | 1.050 | 1.5168 | 64.20 |
| 17 | | ∞ | 0.700 | 1 | - |
| 18 | PRISM EQUIVALENT BLOCK | ∞ | 14.760 | 1.60342 | 38.01 |
| 19 | | ∞ | 0.800 | 1 | - |
| 20 | Filter3 | ∞ | 0.700 | 1.5168 | 64.20 |
| 21 | Filter4 | ∞ | 0.500 | 1.5168 | 64.20 |
| 22 | | ∞ | 0.630 | 1 | - |
| 23 | IMAGING SURFACE | | | | |

| SURFACE NUMBER | +1Dptr | INFINITY | -1Dptr | -4Dptr |
|---|---|---|---|---|
| 11 | 4.750 | 4.283 | 3.808 | 2.500 |
| 13 | 2.700 | 3.167 | 3.642 | 4.950 |

| | |
|---|---|
| L | 49.263 |
| BF(air) | 18.179 |
| BF(air)/L | 0.369 |

# FIG.6

| | | |
|---|---|---|
| $\alpha$ p G1 | TAFD25 | 88 |
| $\alpha$ n G2 | E-FL5 | 91 |
| $\alpha$ n G3 | E-FD8 | 112 |
| $\alpha$ p G4 | PCD51 | 116 |

| | | | |
|---|---|---|---|
| (1) | BF(air)/L | >0.3 | 0.369 |
| (2) | \|1-$\alpha$ p/$\alpha$ n\| G1/G2 | < 20 % | 3.3% |
| | \|1-$\alpha$ p/$\alpha$ n\| G3/G4 | < 20 % | 3.4% |
| (3) | $\Delta$f/$\Delta$F | >0.8 <0.95 | 0.834 |
| (4) | W_2 | < 0.8 | 0.408 |
| (5) | SG2 | < 3.2 | 2.45 |
| (6) | \| 1- IH1(Near)/IH1(Far) \| | < 3% | 2.313 |
| (7) | nd(GN1)/nd(GN2) | <0.95 | 0.936 |
| (8) | nd(GP1) | > 1.85 | 1.904 |
| (9) | nd(GP1)/nd(GN1) | > 1.15 | 1.204 |

# FIG.7

Y-FAN, X-FAN ray aberration plots:

- 1.00 RELATIVE FIELD HEIGHT ( 10.77 )° — 0.02 / -0.02
- 0.54 RELATIVE FIELD HEIGHT ( 5.768 )° — 0.02 / -0.02
- 0.00 RELATIVE FIELD HEIGHT ( 0.000 )° — 0.02 / -0.02

-1 Diopter

RAY ABERRATIONS (MILLIMETERS)

- - - - - 656.2700 NM
——— 587.5600 NM
— — — 435.8300 NM

LONGITUDINAL SPHERICAL ABER.
1.00 / 0.75 / 0.50 / 0.25
-0.050  -0.025  0.0  0.025  0.050
FOCUS (MILLIMETERS)

ASTIGMATIC FIELD CURVES
IMG HT
X / Y
3.68 / 2.76 / 1.84 / 0.92
-0.050  -0.025  0.0  0.025  0.050
FOCUS (MILLIMETERS)

DISTORTION
IMG HT
3.68 / 2.76 / 1.84 / 0.92
-5.0  -2.5  0.0  2.5  5.0
% DISTORTION

-1 Diopter

- - - - - 656.2700 NM
——— 587.5600 NM
— — — 435.8300 NM

EP 4 597 191 A1

# FIG.8

| SURFACE NUMBER | ELEMENT NAME | RADIUS OF CURVATURE | SURFACE INTERVAL | REFRACTIVE INDEX | ABBE NUMBER |
|---|---|---|---|---|---|
| 1 | DIAPHRAGM | ∞ | 1 | 1 | - |
| 2 | Filter1 | ∞ | 1.1 | 1.5168 | 64.20 |
| 3 | | ∞ | 0.543 | 1 | - |
| 4 | G1 | 13.705 | 1.885 | 1.95375 | 32.32 |
| 5 | G2 | -22.410 | 0.876 | 1.54814 | 45.82 |
| 6 | | 8.145 | 3.260 | 1 | - |
| 7 | G3 | -6.318 | 2.500 | 1.72825 | 28.32 |
| 8 | G4 | 35.750 | 4.000 | 1.59349 | 67.00 |
| 9 | | -9.260 | 0.300 | 1 | - |
| 10 | G5 | -45.834 | 1.998 | 1.59349 | 67.00 |
| 11 | | -21.075 | 4.594 | 1 | - |
| 12 | G6 | 21.815 | 2.335 | 1.59349 | 67.00 |
| 13 | | -82.345 | 2.700 | 1 | - |
| 14 | | ∞ | 1.000 | 1 | - |
| 15 | | ∞ | 1.660 | 1 | - |
| 16 | Filter2 | ∞ | 1.050 | 1.5168 | 64.20 |
| 17 | | ∞ | 0.700 | 1 | - |
| 18 | PRISM EQUIVALENT BLOCK | ∞ | 14.760 | 1.60342 | 38.01 |
| 19 | | ∞ | 0.800 | 1 | - |
| 20 | Filter3 | ∞ | 0.700 | 1.5168 | 64.20 |
| 21 | Filter4 | ∞ | 0.500 | 1.5168 | 64.20 |
| 22 | | ∞ | 0.630 | 1 | - |
| 23 | IMAGING SURFACE | | | | |

| SURFACE NUMBER | +1Dptr | INFINITY | -1Dptr | -4Dptr |
|---|---|---|---|---|
| 11 | 4.594 | 4.162 | 3.737 | 2.500 |
| 13 | 2.700 | 3.132 | 3.557 | 4.794 |

| L | 48.892 |
|---|---|
| BF(air) | 18.179 |
| BF(air)/L | 0.372 |

# FIG.9

| $\alpha$p G1 | TAFD45 | 85 |
|---|---|---|
| $\alpha$n G2 | E-FEL1 | 96 |
| $\alpha$n G3 | E-FD10 | 109 |
| $\alpha$p G4 | PCD51 | 116 |

| (1) | BF(air)/L | >0.3 | 0.372 |
|---|---|---|---|
| (2) | \|1-$\alpha$p/$\alpha$n\| G1/G2 | < 20 % | 11.5% |
|  | \|1-$\alpha$p/$\alpha$n\| G3/G4 | < 20 % | 6.0% |
| (3) | $\Delta$f/$\Delta$F | >0.8 <0.95 | 0.905 |
| (4) | W_2 | < 0.8 | 0.517 |
| (5) | SG2 | < 3.2 | 3.14 |
| (6) | \| 1- IH1(Near)/IH1(Far) \| | < 3% | 1.696 |
| (7) | nd(GN1)/nd(GN2) | <0.95 | 0.896 |
| (8) | nd(GP1) | > 1.85 | 1.954 |
| (9) | nd(GP1)/nd(GN1) | > 1.15 | 1.262 |

# FIG.10

Y-FAN     1.00 RELATIVE     X-FAN
FIELD HEIGHT
( 10.78 )°
0.02                 0.02

-0.02                 -0.02

0.54 RELATIVE
FIELD HEIGHT
( 5.768 )°
0.02                 0.02

-0.02                 -0.02

0.00 RELATIVE
FIELD HEIGHT
( 0.000 )°
0.02                 0.02

-0.02                 -0.02

-1 Diopter

RAY ABERRATIONS
(MILLIMETERS)

– – – – – 656.2700 NM
———— 587.5600 NM
– – – 435.8300 NM

– – – – – 656.2700 NM
———— 587.5600 NM
– – – 435.8300 NM

LONGITUDINAL
SPHERICAL ABER.

ASTIGMATIC
FIELD CURVES

DISTORTION

IMG HT
1.00

0.75

0.50

0.25

IMG HT
3.68 X Y

2.76

1.84

0.92

IMG HT
3.68

2.76

1.84

0.92

-0.050    0.0    0.050
-0.025    0.025
FOCUS (MILLIMETERS)

-0.050    0.0    0.050
-0.025    0.025
FOCUS (MILLIMETERS)

-5.0 -2.5   0.0   2.5   5.0
% DISTORTION

-1 Diopter

EP 4 597 191 A1

# FIG.11

| SURFACE NUMBER | ELEMENT NAME | RADIUS OF CURVATURE | SURFACE INTERVAL | REFRACTIVE INDEX | ABBE NUMBER |
|---|---|---|---|---|---|
| 1 | DIAPHRAGM | ∞ | 1 | 1 | - |
| 2 | Filter1 | ∞ | 1.1 | 1.5168 | 64.20 |
| 3 | | ∞ | 0.543 | 1 | - |
| 4 | G1 | 12.950 | 1.900 | 1.90366 | 31.32 |
| 5 | G2 | -22.410 | 0.600 | 1.58144 | 40.89 |
| 6 | | 8.220 | 4.070 | 1 | - |
| 7 | G3 | -5.900 | 2.500 | 1.68893 | 31.16 |
| 8 | G4 | 35.750 | 3.490 | 1.59349 | 67.00 |
| 9 | | -8.470 | 0.300 | 1 | - |
| 10 | G5 | -196.59 | 2.140 | 1.59349 | 67.00 |
| 11 | | -25.390 | 4.750 | 1 | - |
| 12 | G6 | 20.410 | 2.370 | 1.48749 | 70.44 |
| 13 | | -92.790 | 2.700 | 1 | - |
| 14 | | ∞ | 1.000 | 1 | - |
| 15 | | ∞ | 1.660 | 1 | - |
| 16 | Filter2 | ∞ | 1.050 | 1.5168 | 64.20 |
| 17 | | ∞ | 0.700 | 1 | - |
| 18 | PRISM EQUIVALENT BLOCK | ∞ | 14.760 | 1.60342 | 38.01 |
| 19 | | ∞ | 0.800 | 1 | - |
| 20 | Filter3 | ∞ | 0.700 | 1.5168 | 64.20 |
| 21 | Filter4 | ∞ | 0.500 | 1.5168 | 64.20 |
| 22 | | ∞ | 0.630 | 1 | - |
| 23 | IMAGING SURFACE | | | | |

| SURFACE NUMBER | +1Dptr | INFINITY | -1Dptr | -4Dptr |
|---|---|---|---|---|
| 11 | 4.750 | 4.283 | 3.808 | 2.500 |
| 13 | 2.700 | 3.167 | 3.642 | 4.950 |

| | |
|---|---|
| L | 49.263 |
| BF(air) | 18.179 |
| BF(air)/L | 0.369 |

# FIG.12

| $\alpha$p G1 | TAFD25 | 83 |
|---|---|---|
| $\alpha$n G2 | E-FL5 | 102 |
| $\alpha$n G3 | E-FD8 | 109 |
| $\alpha$p G4 | PCD51 | 116 |

| (1) | BF(air)/L | >0.3 | 0.369 |
|---|---|---|---|
| (2) | \|1-$\alpha$p/$\alpha$n\| G1/G2 | < 20 % | 18.6% |
| | \|1-$\alpha$p/$\alpha$n\| G3/G4 | < 20 % | 6.0% |
| (3) | Δf/ΔF | >0.8 <0.95 | 0.872 |
| (4) | W_2 | < 0.8 | 0.422 |
| (5) | SG2 | < 3.2 | 2.45 |
| (6) | \| 1- IH1(Near)/IH1(Far) \| | < 3% | 1.969 |
| (7) | nd(GN1)/nd(GN2) | <0.95 | 0.866 |
| (8) | nd(GP1) | > 1.85 | 1.881 |
| (9) | nd(GP1)/nd(GN1) | > 1.15 | 1.265 |

# FIG.13

1.00 RELATIVE FIELD HEIGHT ( 10.77 )°

Y-FAN    X-FAN

0.54 RELATIVE FIELD HEIGHT ( 5.767 )°

0.00 RELATIVE FIELD HEIGHT ( 0.000 )°

-1Diopter

RAY ABERRATIONS (MILLIMETERS)

- - - - - - 656.2700 NM
————— 587.5600 NM
— — — 435.8300 NM

- - - - - - 656.2700 NM
————— 587.5600 NM
— — — 435.8300 NM

LONGITUDINAL SPHERICAL ABER.

ASTIGMATIC FIELD CURVES

DISTORTION

FOCUS (MILLIMETERS)

FOCUS (MILLIMETERS)

% DISTORTION

-1Diopter

EP 4 597 191 A1

# FIG.14

| SURFACE NUMBER | ELEMENT NAME | RADIUS OF CURVATURE | SURFACE INTERVAL | REFRACTIVE INDEX | ABBE NUMBER |
|---|---|---|---|---|---|
| 1 | DIAPHRAGM | ∞ | 1 | 1 | - |
| 2 | Filter1 | ∞ | 1.1 | 1.5168 | 64.20 |
| 3 | | ∞ | 0.543 | 1 | - |
| 4 | G1 | 12.811 | 1.916 | 1.95375 | 32.32 |
| 5 | G2 | -22.410 | 0.642 | 1.54814 | 45.82 |
| 6 | | 7.623 | 3.309 | 1 | - |
| 7 | G3 | -6.150 | 2.500 | 1.72825 | 28.32 |
| 8 | G4 | 35.750 | 4.000 | 1.59349 | 67.00 |
| 9 | | -9.059 | 0.300 | 1 | - |
| 10 | G5 | -43.036 | 1.982 | 1.59349 | 67.00 |
| 11 | | -20.919 | 4.577 | 1 | - |
| 12 | G6 | 21.978 | 3.000 | 1.59349 | 67.00 |
| 13 | | -77.918 | 2.700 | 1 | - |
| 14 | | ∞ | 1.000 | 1 | - |
| 15 | | ∞ | 1.660 | 1 | - |
| 16 | Filter2 | ∞ | 1.050 | 1.5168 | 64.20 |
| 17 | | ∞ | 0.700 | 1 | - |
| 18 | PRISM EQUIVALENT BLOCK | ∞ | 14.760 | 1.60342 | 38.01 |
| 19 | | ∞ | 0.800 | 1 | - |
| 20 | Filter3 | ∞ | 0.700 | 1.5168 | 64.20 |
| 21 | Filter4 | ∞ | 0.500 | 1.5168 | 64.20 |
| 22 | | ∞ | 0.630 | 1 | - |
| 23 | IMAGING SURFACE | | | | |

| SURFACE NUMBER | +1Dptr | INFINITY | -1Dptr | -4Dptr |
|---|---|---|---|---|
| 11 | 4.577 | 4.150 | 3.728 | 2.500 |
| 13 | 2.700 | 3.128 | 3.549 | 4.777 |

| | |
|---|---|
| L | 49.369 |
| BF(air) | 18.179 |
| BF(air)/L | 0.368 |

# FIG.15

| | | |
|---|---|---|
| $\alpha$ p G1 | TAFD45 | 85 |
| $\alpha$ n G2 | E-FEL1 | 96 |
| $\alpha$ n G3 | E-FD10 | 109 |
| $\alpha$ p G4 | PCD51 | 116 |

| | | | |
|---|---|---|---|
| (1) | BF(air)/L | >0.3 | 0.368 |
| (2) | \|1-$\alpha$p/$\alpha$n\| G1/G2 | < 20 % | 11.5% |
| | \|1-$\alpha$p/$\alpha$n\| G3/G4 | < 20 % | 6.0% |
| (3) | $\Delta$f/$\Delta$F | >0.8 <0.95 | 0.912 |
| (4) | W_2 | < 0.8 | 0.692 |
| (5) | SG2 | < 3.2 | 3.14 |
| (6) | \| 1- IH1(Near)/IH1(Far) \| | < 3% | 1.593 |
| (7) | nd(GN1)/nd(GN2) | <0.95 | 0.896 |
| (8) | nd(GP1) | > 1.85 | 1.954 |
| (9) | nd(GP1)/nd(GN1) | > 1.15 | 1.262 |

# FIG.16

Y-FAN    1.00 RELATIVE
FIELD HEIGHT    X-FAN
0.02    ( 10.78 )°    0.02

-0.02    -0.02

0.54 RELATIVE
0.02    FIELD HEIGHT    0.02
( 5.768 )°

-0.02    -0.02

0.00 RELATIVE
0.02    FIELD HEIGHT    0.02
( 0.000 )°

-0.02    -0.02

-1Diopter

RAY ABERRATIONS
(MILLIMETERS)

- - - - - - 656.2700 NM
——————— 587.5600 NM
— — — 435.8300 NM

- - - - - - 656.2700 NM
——————— 587.5600 NM
— — — 435.8300 NM

LONGITUDINAL
SPHERICAL ABER.

1.00

0.75

0.50

0.25

-0.050    0.050
-0.025    0.0    0.025
FOCUS (MILLIMETERS)

ASTIGMATIC
FIELD CURVES

IMG HT
3.68    X    Y

2.76

1.84

0.92

-0.050    0.050
-0.025    0.0    0.025
FOCUS (MILLIMETERS)

DISTORTION

IMG HT
3.68

2.76

1.84

0.92

-5.0  -2.5  0.0  2.5  5.0
% DISTORTION

-1Diopter

EP 4 597 191 A1

# FIG.17

EP 4 597 191 A1

# FIG.18

## INTERNATIONAL SEARCH REPORT

| | International application No. |
|---|---|
| | **PCT/JP2023/033668** |

**A. CLASSIFICATION OF SUBJECT MATTER**

***G02B 13/00***(2006.01)i; ***A61B 1/00***(2006.01)i; ***G02B 13/16***(2006.01)i; ***G02B 23/26***(2006.01)i
FI:   G02B13/00; A61B1/00 R; A61B1/00 511; A61B1/00 735; G02B13/16; G02B23/26 A

According to International Patent Classification (IPC) or to both national classification and IPC

**B. FIELDS SEARCHED**

Minimum documentation searched (classification system followed by classification symbols)

G02B13/00; A61B1/00; G02B13/16; G02B23/26

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2021-4998 A (CANON INC.) 14 January 2021 (2021-01-14)<br>examples 1-4 | 1-13 |
| A | WO 2019/187874 A1 (SONY CORP.) 03 October 2019 (2019-10-03)<br>examples 1-5 | 1-13 |
| A | JP 6147455 B1 (OLYMPUS CORP.) 26 May 2017 (2017-05-26)<br>examples 1-3 | 1-13 |

| ☐ Further documents are listed in the continuation of Box C. | ☑ See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **26 October 2023** | **07 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/033668**

| Patent document cited in search report | | | Publication date (day/month/year) | Patent family member(s) | | | Publication date (day/month/year) |
|---|---|---|---|---|---|---|---|
| JP | 2021-4998 | A | 14 January 2021 | (Family: none) | | | |
| WO | 2019/187874 | A1 | 03 October 2019 | US | 2021/0030263 | A1 | |
| | | | | examples 1-5 | | | |
| | | | | EP | 3751325 | A1 | |
| | | | | CN | 111971604 | A | |
| JP | 6147455 | B1 | 26 May 2017 | US | 2017/0354321 | A1 | |
| | | | | examples 1-3 | | | |
| | | | | EP | 3329832 | A1 | |
| | | | | CN | 107405058 | A | |

Form PCT/ISA/210 (patent family annex) (January 2015)

**EP 4 597 191 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019187874 A